Europäisches Patentamt

(19) European Patent Office

Office européen des brevets

(11) Publication number: **0 214 803**
A2

(12) # EUROPEAN PATENT APPLICATION

(21) Application number: 86306562.9

(22) Date of filing: 26.08.86

(51) Int. Cl.⁴: **A 61 M 1/34**

(30) Priority: 03.09.85 GB 8521867

(43) Date of publication of application:
18.03.87 Bulletin 87/12

(84) Designated Contracting States:
AT BE CH DE IT LI LU NL SE

(71) Applicant: FISONS plc
Fison House Princes Street
Ipswich Suffolk IP1 1QH(GB)

(72) Inventor: Burrett, Kenneth Frederick
23 Montrouge Crescent
Epsom Downs Surrey, KP17 3PB(GB)

(72) Inventor: Molloy, James Oscar
5 The Crescent
Belmont Surrey, SM2 6PB(GB)

(72) Inventor: Royffe, David George
211B Upper Richmond Road
London SW15 6SQ(GB)

(72) Inventor: Waters, Anthony Paul Charles
18 Fairfield Avenue
Datchet Berkshire SL3 9NQ(GB)

(72) Inventor: Wrigg, James
10 The Pines
Forest Fields Haywards Heath RH16 3TX(GB)

(74) Representative: Craig, Christopher Bradberry et al,
Fisons plc 12 Derby Road
Loughborough Leicestershire LE11 0BB(GB)

(54) Blood fractionation system, apparatus and pathway and method of processing blood.

(57) There is described a blood fractionation system, apparatus and method of processing blood.

There is also described a blood fractionation pathway (1 - 15) for use with a centrifugal blood fractionation apparatus (16 - 31) and preferably including a substantially toroidal separation chamber (1).

EP 0 214 803 A2

Croydon Printing Company Ltd.

./...

Fig.1.

Blood Fractionation System, Apparatus and Pathway and
Method of Processing Blood

This invention relates to a blood fractionation
system, a blood fractionation apparatus and a blood
fractionation pathway, and methods of processing blood.

Several methods and apparatus have been developed for
the in-vivo processing of whole blood, in which whole
blood is taken from a donor, a desired blood component
separated and collected and the processed blood returned
to the donor. One such technique is plasmapheresis, in
which whole blood is separated into a plasma component and
a cellular component.

In-vivo blood processing apparatus is usually used in
association with a disposable fractionation pathway, which
includes a separation chamber, first and second blood
component collection chambers, first and second blood
component conduits for establishing fluid communication
between the separation chamber and the first and second
blood component collection chambers respectively, and a
donor conduit capable of establishing fluid communication
between a donor and the separation chamber.

In-vivo blood processing apparatus may be of the
centrifugal type, in which the differing density of the
collected blood component causes the components to
congregate for collection at particular radial distances
in a centrifuge, or may be of the filter type, in which the particle size of the collected component allows only that component to pass through a filter membrane into a collection chamber.

Filter type apparatus suffer from the disadvantage that the collected blood component is exposed to high shear rates on filtration, which can lead to protein degradation and loss of protein activity. In addition the filtration chamber is expensive, leading to a high cost for the disposable pathway.

Centrifuge apparatus suffer from the disadvantage that during blood processing, at high rotational speeds the flow of blood components along the first and second exit conduits can be impeded by the build-up of air locks in the conduits. This problem of air locks is a difficulty common to most continuous flow centrifuges, eg centrifuges with either rotating seals or seal-less systems. In many blood processing methods, the maintenance of differential flow rates in the conduits is an integral part of the separation process, so that the build up of air locks causes the separation process to fail.

A further disadvantage of disposable pathways designed for use in a centrifuge apparatus and having a rotating seal is that either the seal is very expensive and/or the seal is unreliable.

We have now found a blood component fractionation system, a blood component fractionation pathway, a blood fractionation apparatus and methods for fractionating blood which either overcome or substantially mitigate these disadvantages.

According to the invention there is provided a blood component fractionation system comprising

a centrifugal rotor chamber,

a separation chamber adapted for placement within the rotor chamber

first and second blood component collection chambers,

first and second blood component conduits for establishing fluid communication between the separation chamber and the first and the second blood component collection chambers respectively,

a donor conduit capable of establishing fluid communication between a donor and the separation chamber,

a return conduit for establishing fluid communication between one of the blood component conduits and the donor conduit,

a donor pump capable of pumping fluid along the donor conduit,

a blood component pump capable of pumping fluid along one of the blood component conduits, and system control means including means responsive to the weight of fluid in each of the first and second blood component collection chambers, the system control means being adapted to control the system during an operating sequence comprising at least one primary blood component collection phase during which blood is drawn from a donor, the collection phase comprising at least one blood component collection cycyle during which blood is continuously drawn from the donor and first blood component is conveyed along its respective blood component conduit whilst flow along the second blood component conduit is stopped, and then the second blood component is conveyed along its respective blood component conduit whilst flow along the first blood component conduit is stopped, such that in operation flow of fluid along the first blood component conduit is stopped, and flow of fluid along the second blood component conduit initiated, by the collected first blood component increasing by a pre-determined weight, and flow of fluid along the second blood component conduit is stopped, and flow of fluid along the first blood component conduit initiated, by the collected second blood component increasing by a pre-determined weight.

The relative amount of fluid conveyed along each of the blood component conduits depends, inter alia, on the dimensions of the conduit, the bulk properties of the fluid, the nature of the means for conveyancing the fluid and the relative period of time for which flow takes place in each conduit.

The pre-determined weight of collected first blood component to the pre-determined weight of collected second blood component is preferably from 1.7:1 to 2.3:1.

The pre-determined weight of collected first blood component is preferably from about 0.1 grams, eg 0.5 to 5 grams blood.

Preferably the first blood component is a cellular component and the second blood component is a plasma component.

The primary blood component collection phase may also include a reinfusion stage comprising returning first blood component from its respective blood component collection chamber to the donor, the reinfusion stage being initiated by one of the collected first and second blood components attaining a pre-determined weight.

The operating sequence may also include a secondary blood component collection phase comprising displacement of second blood component from the separation chamber along its respective blood component conduit by returning first blood component from its respective blood component collection chamber to the separation chamber, the secondary blood component collection phase being initiated by the collected second blood component attaining a pre-determined weight.

The operating sequence may further include a final reinfusion phase comprising conveying the contents of the separation chamber to the first blood component collection chamber and then returning the contents of the first blood component collection chamber to the donor, the final reinfusion phase being initiated by the collected second blood component attaining a pre-determined weight.

The final reinfusion phase may also be terminated by the first collected blood component attaining a pre-determined weight.

The means responsive to the weight of fluid in each of the first and second blood component collection chambers preferably includes electronic weight transducers.

The system control means includes preferably means responsive to the presence or absence of fluid in the first blood component conduit, the second blood component conduit and/or the return conduit.

The primary blood component collection phase is preferably initiated by the detection of fluid in one of the first and second blood component conduits.

When the operating sequence includes a final reinfusion phase, that phase is preferably terminated by the detection of the absence of fluid in the return conduit.

The means responsive to the presence or absence of fluid preferably includes optical or ultrasonic transducers.

We prefer the blood fractionation system to comprise a disposable blood fractionation pathway and a blood fractionation apparatus. In particular, we prefer the separation chamber, the first and second blood component collection chambers, the first and second blood component conduits, the donor conduit and the return conduit together to constitute a disposable blood fractionation pathway adapted for use with a blood fractionation apparatus including the cetrifugal rotor chamber, the donor pump, the blood component pump and the system control means.

According to the invention there is also provided a blood fractionation pathway, for use in association with a blood fractionation apparatus including a centrifugal separation chamber, the pathway comprising a separation chamber adapted for placement within the rotor chamber of the blood fractionation apparatus,

first and second blood component collection chambers,

first and second blood component conduits for establishing fluid communication between the separation chamber and the first and second blood component collection chambers respectively,

a donor conduit capable of establishing fluid communication between a donor and the separation chamber,

and a return conduit for establishing fluid communication between one of the blood component collection conduits and the donor conduit.

The donor conduit and first and second blood component conduits may communicate with the separation chamber by means of a rotatable seal. However, we prefer the conduits to be adapted for use with a blood processing device in which no rotating seal is required, eg devices of a known type in which the centrifugal separation chamber is rotated with twice the angular velocity of a chamber support frame to which the conduits are attached.

The separation chamber may comprise an assembly of two or more containers which are adapted to be symmetrically disposed about the axis of centrifugation. However, we prefer the separation chamber to be a single container which is substantially toroidal in shape. A toroidal container may be made, for example from a length of tube sealed at each end. We particularly prefer the donor conduit to communicate with one end of the tube and the first and second blood component conduits to communicate with the opposite end of the tube.

In a particularly preferred embodiment, the separation chamber is provided on its internal surface with a plurality of longitudinal ribs. Two of said ribs are preferably located adjacent to the orifice of the first or second blood component comduit and are of substantially greater depth than the remaining ribs.

For centrifugal separation to occur, the separation chamber is provided with container walls which in use are radially separated. Each of the blood component conduits terminates within the separation chamber. We prefer one of the blood component conduits to terminate immediately adjacent to the inner radial wall. This enables the light fraction (eg plasma) of whole blood to be collected. We particularly prefer this conduit to terminate within a small blister or lagoon, which projects from the inner radial wall of the container towards the axis of centrifugation. The other blood component conduit preferably terminates immediately adjacent to the outer radial wall of the separation chamber. This enables the heavy fraction (eg rich in red blood cells) to be collected.

We prefer the return conduit to establish fluid communication between the heavy fraction blood component conduit and the donor conduit.

We prefer the first and second blood component conduits and the donor conduit to be connected to the separation chamber by nipples. We particularly prefer that blood component conduit which terminates immediately adjacent to the inner radial wall of the separation chamber to be connected to the separation chamber by means of a nipple with a substantially frusto-conical orifice.

The donor conduit is preferably provided with a pressure pouch or "mouse", which is capable of actuating a pressure sensor provided on the blood fractionation apparatus. Such a sensor may be used to monitor both the loading pressure, when whole blood is withdrawn from the donor, and return pressure, when blood component, eg an enriched red blood cell component, is returned to the donor. The pressure pouch is preferably located on the donor conduit between the junction of the return conduit with the donor conduit and the point of attachment of the donor conduit to the donor.

The donor conduit may also be provided with a filter pouch. The filter pouch is preferably located on the donor conduit between the junction of the return conduit with the donor conduit and the point of attachment of the donor conduit to the donor.

When the separation chamber comprises a tube sealed at both ends, donor conduit is preferably connected to the separation chamber at the opposite end of the separation chamber to the end at which the first and second blood component conduits are connected to the separation chamber.

We prefer a portion of the pathway including the portion which undergoes angular motion to be made of torsion-stiff, but flexible material, eg nylon. The remainder of the pathway may be made of compliant material, eg polyvinylchloride. Surprisingly we have found that good leakproof seals can be made between the torsion-stiff and the compliant materials using cyanoacrylate adhesives.

The blood component fractionation system is preferably provided with an anticoagulant container and an anticoagulant conduit for establishing fluid communication between the anticoagulant container and the donor conduit.

The anticoagulant conduit is preferably of sufficient length that a portion of the conduit is adapted to make an operative contact with fluid conveyancing means, eg a peristaltic pump.

According to the invention there is also provided a method of fractionating blood comprising separating the blood into first and second blood components in a separation chamber and collecting said first and second blood components in at least one collection cycle during which blood is continuously drawn from a donor, the collection cycle comprising conveying first one and then the other blood component to first and second blood component collection chambers respectively.

Preferably upon termination of the collection of said first and second blood components, one of said first and second components is returned to the donor.

According to the invention there is also provided a method of fractionating blood comprising separating the blood into first and second blood components in a separation chamber and collecting said first and second blood components in first and second blood component collection chambers respectively, wherein the method includes a phase during which no blood is drawn from a donor and one of said first and second blood components is collected by displacing it from the separation chamber by returning the other one of said first and second blood components from its respective blood component collection chamber to the separation chamber.

When the fractionation method is plasmapheresis, we prefer the blood component returned to the separation chamber to be the cellular component and the component displaced from the separation chamber to be plasma.

According to the invention there is also provided a method of fractionating blood comprising separating the blood into first and second blood components in a separation chamber and collecting said first and second blood components in first and second blood component collection chamber respectively, wherein the method includes a final reinfusion phase in which the contents of the separation chamber are conveyed to one of the first and second blood component collection chambers and the contents of the blood component collection chamber are then returned to the donor.

According to a further aspect of the invention there is provided a method of fractionating blood comprising at least one primary blood component collection phase during which blood is drawn from a donor and separated into first and second blood components in a separation chamber, a secondary blood component collection phase during which no blood is drawn from the donor, and a final reinfusion phase, wherein

said primary blood component collection phase comprises at least one blood component collection cycle during which blood is continuously drawn from the donor and a reinfusion stage, the blood component collection cycle comprising conveying first one and then the other blood component to the first and second blood component collection chambers respectively, and the reinfusion stage comprising returning the first blood component from its respective blood component collection chamber to the donor,

said secondary blood component collection phase comprises displacement of second blood component from the separation chamber to its respective blood component collection chamber by returning first blood component from its respective blood component collection chamber to the separation chamber, and

said final reinfusion phase comprises conveying the contents of the separation chamber to the first blood component collection chamber and then returning the contents of the first blood component collection chamber to the donor.

Where the blood fractionation system comprises a blood fractionation pathway and a blood fractionation apparatus, the blood fractionation apparatus preferably includes means for conveying fluid to and from a donor, eg a peristaltic pump capable of moving both forwards and backwards. The apparatus preferably includes a further means for conveying fluid, for conveying one of the fractionated blood components to its blood component collection chamber. The apparatus is preferably a centrifugal fractionation apparatus, provided with a centrifuge rotor. More preferably, the apparatus is provided with means for rotating a looped portion of the donor conduit of a pathway at half the angular velocity of the rotor chamber, such that continuous separation of blood can be effected without the need for rotating seals.

When the pathway for use in association with the apparatus includes an anticoagulant chamber, the apparatus is preferably provided with means for conveying anticoagulant, eg a peristaltic pump.

The apparatus is preferably provided with means responsive to a pre-determined maximum weight of fluid in one of the blood component collection chambers. The apparatus is more preferably provided with means responsive to pre-determined weights of fluid in each of the blood component collection chambers.

The means respsonsive to the weight of fluid in one or each of the blood component collection chambers may include an electronic weight transducer.

The apparatus may further be provided with pressure response means responsive to a pressure pouch located on the pathway. The pressure response means is preferably capable of responding to relatively high donation pressures as well as low return pressures. The pressure response means is preferably operably connected to system control means capable of terminating a fractionation procedure if certain pre-determined operating criteria are not met.

A preferred embodiment of the invention will now be illustrated with reference to the accompanying drawings in which

fig 1 is a diagrammatic representation of a blood component fractionation system including a separation chamber 1, and

fig 2 is a cross-section through the separation chamber along the line II - II' in figure 1.

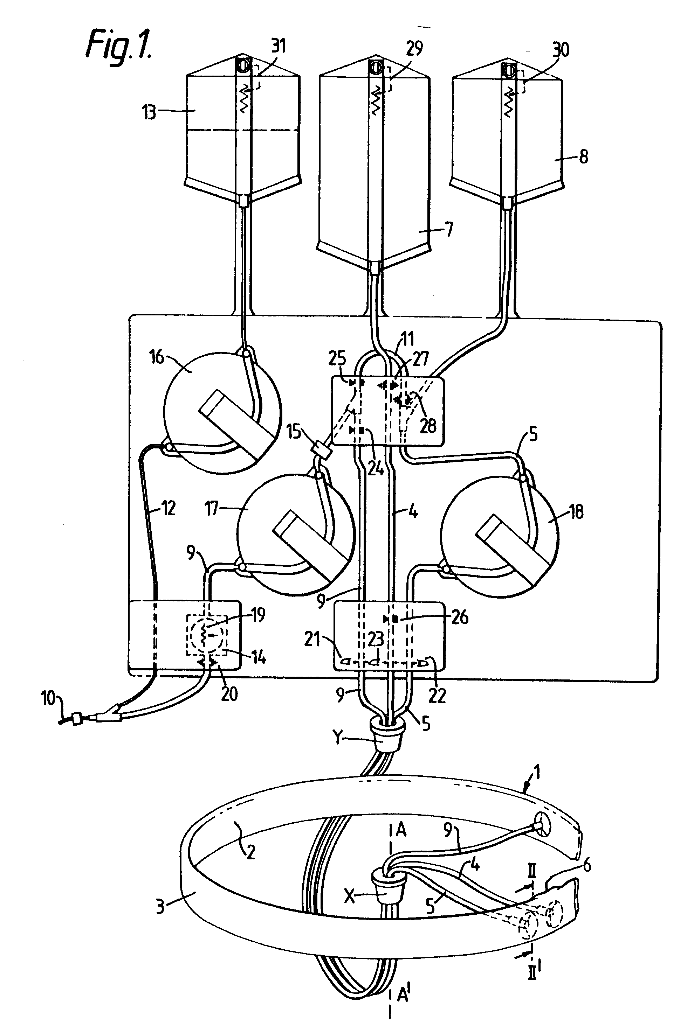

Referring first to figure 1, a blood fractionation system comprises a blood fractionation pathway and an extracorporeal blood fractionation apparatus having a centrifugal rotor chamber. The blood fractionation pathway is a disposable unit made substantially of plastics material and adapted for easy mounting on the blood fractionation apparatus. The pathway includes a separation chamber 1 formed from a tube arranged in the general shape of a toroid and sealed at each end. The separation chamber is adapted for placement in the centrifugal rotor chamber of the blood fractionation apparatus.

The separation chamber 1 is provided with inner and out chamber walls, 2 and 3 respectively, which in use are radially separated. The separation chamber 1 is provided at one end with a first blood component conduit 4 (the plasma conduit) and a second blood component conduit 5 (the red blood cell conduit). The plasma conduit 4 terminates within the separation chamber 1 immediately adjacent to the inner wall 2 within a small blister 6 in the inner wall which protrudes towards the axis of centrifugation. The plasma conduit 4 connects the separation chamber 1 with a first blood component collection chamber (plasma container) 7.

The red blood cell conduit 5 terminates within the separation chamber 1 immediately adjacent to the outer wall 3 and connects the separation chamber 1 with a second blood component collection chamber (red blood cell reservoir) 8.

The separation chamber 1 is further provided, at its other closed end portion, with a donor conduit 9 which joins the separation chamber 1 with a donor, eg via a phlebotomy needle 10.

A return conduit 11 is provided which establishes fluid communication between the red blood cell conduit 5 and the donor conduit 9.

The donor conduit 9 is further provided with an anticoagulant conduit 12 which establishes communication between the donor conduit 9 and an anticoagulant chamber 13.

The donor conduit 9 is provided with a pressure pouch or "mouse" 14 which is located between the junction of the anticoagulant conduit 12 with the donor conduit 9 and the junction of the return conduit 11 with the donor conduit 9. The donor conduit is further provided with a filter 15.

The blood component fractionation apparatus includes an anticoagulant peristaltic pump 16, a blood peristaltic pump 17, a red cell peristaltic pump 18, a pressure sensor 19, a donor conduit ultrasonic transducer 20, a blood optical transducer 21, a red cell optical transducer 22, a plasma optical transducer 23, a donor conduit clamp 24, a return conduit clamp 25, a plasma conduit clamp 26, a plasma reservoir ultrasonic transducer 27 and a return conduit ultrasonic transducer 28. The apparatus is further provided with a first weight transducer 29 adapted to respond to the weight of plasma chamber 7, a second weight transducer 30 adapted to respond to the weight of red cell component chamber 8 and a third weight transducer 31 adapted to respond to the weight of anticoagulant chamber 13. Components 16 - 31 are arranged to operatively interact with the blood fractionation pathway in a manner which will become clear from the following description.

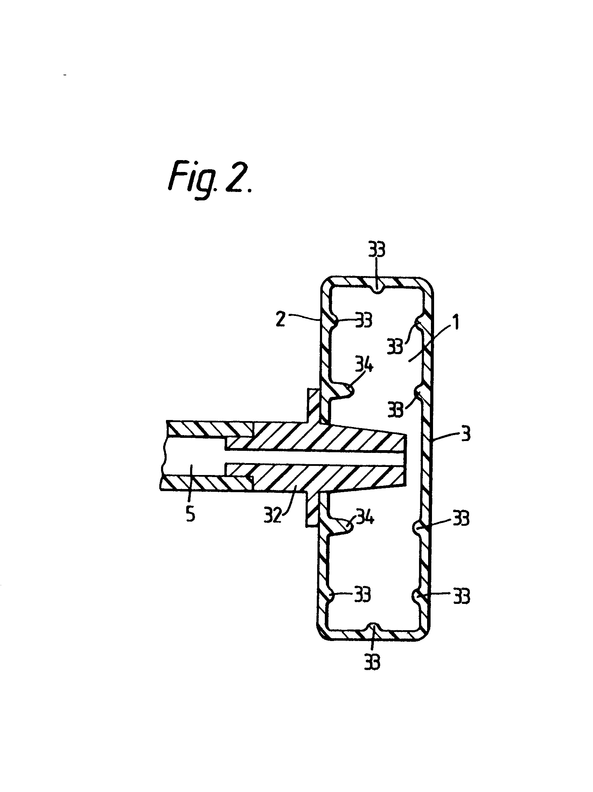

Referring now to figure 2, the red blood cell conduit 5 is connected to the separation chamber 1 by means of nipple 32 such that the conduit 5 terminates within the chamber 1 close to the outer chamber wall 3. The separation chamber 1 is provided on its interior surface with a plurality of longitudinal ribs 33, 34. The two ribs 34 adjacent to the nipple 32 are of substantially greater depth than the remaining ribs 33. In an alternative embodiment, which is not illustrated, the separation chamber 1 may be provided with two ribs of substantially greater depth than the remaining ribs 33 in the wall 3 opposing the nipple 32.

The blood fractionation pathway is installed in the blood fractionation apparatus, with the separation chamber 1 radially arranged around the axis of rotation of the rotor in the general form of a toroid. The donor 9, plasma 4 and red cell 5 conduits pass along the axis of centrifugation A-A' and are fixed at a lower fixed point X, folded underneath the separation chamber 1, looped around the outer wall 3 and attached to an upper fixed point Y which also lies on the axis A-A'. The looped portions of conduits 9, 4 and 5 are arranged to rotate with one half the angular velocity of the centrifuge rotor and the associated separation chamber 1. This obviates the need for a rotating seal and permits rotation of the separation chamber 1 without tangling of the conduits.

The donor conduit 9 interacts with the blood fractionation apparatus in the following way: beginning from the fixed point Y, the donor conduit 9 passes through the blood optical detector 21, then passes through the clamp 24, the filter 15 and pump 17 before coming to pressure pouch 14 which is located so that it is capable of actuating the pressure sensor 19. The donor conduit then passes through the ultrasonic transducer 20 and thence to the phlebotomy needle 10.

The red blood cell conduit 5 interacts with the blood fractionation apparatus in the following way: beginning from the fixed point Y, the red blood cell conduit 5 passes through the red cell optical transducer 22 and the red cell pump 18 and after the junction of the return conduit 11 with the red blood cell conduit 5 terminates in the red cell reservoir 8.

The plasma conduit 4 interacts with the blood fractionation apparatus in the following way: beginning from the fixed point Y, the plasma conduit 4 passes through the plasma optical transducer 23, plasma conduit clamp 26 and plasma conduit ultrasonic detector 27 before terminating in plasma container 7.

Between its junction with the red cell conduit 5 and its junction with the donor conduit 9, the return conduit 11 passes through the return conduit ultrasonic detector 28 and the return conduit clamp 25.

The anticoagulant conduit 12 interacts with the anticoagulant pump 16.

The system is used to collect blood components as follows:

a) After the blood fractionation pathway has been installed in the blood fractionation apparatus, the system is primed with anticoagulant solution from the anticoagulant container 13 by actuation of pump 16. Priming is complete when a pressure rise is detected by the pressure sensor 19.

b)    The system is then connected to the donor by the phlebotomy needle 10. The blood pump 17 and the anticoagulant pump 16 operate together to allow controlled flow of anticoagulated blood from the donor. The flow rate is controlled with reference to the fullness of the pressure pouch 14 and the pumps 17 and 16 slow if the flow of blood from the donor reduces so that the draw rate never exceeds the donor bleeding rate. The speed of the pump 16 is adjusted so that anticoagulant is delivered at about 8% of the blood flow rate. Typically the blood flow rate is about 65 ml/min. During this phase of operation the return conduit clamp 25 is closed and the clamps 24 and 26 are open.

c)    Once blood reaches the separation chamber 1 (detected by the blood conduit optical transducer 21) the centrifuge rotor starts to spin. The pumps 16 and 17 continue to deliver anticoagulated blood into the separation chamber 1 where the centrifugal field concentrates the red cell component towards the outer surface 3.

d)    When red cells are detected by optical transducer 22, the separation chamber 1 is full; clamp 24 is closed and clamp 25 opened to flush any air from the loop of the return conduit 11.

(e) With clamp 24 open and 25 and 26 closed, the red cell pump 18 is actuated to fill the red cell conduit 5. When the weight transducer 30 has detected about 5g of red cells in chamber 8, the rotor is accelerated to about 1800 rpm.

(f) Plasma and red cell components are then collected by alternately conveying fluid along plasma conduit 4 then red cell conduit 5. This is achieved by running red cell pump 18 with clamp 26 closed until a pre-determined amount of red cells, eg 2-5g, is detected by transducer 30, then red cell pump 18 is stopped, clamp 26 opened and plasma collected in chamber 7 until a pre-determined amount of plasma is detected by transducer 29.

(g) This alternating collection cycle is repeated until the weight of red cell chamber 8 exceeds a pre-determined maximum weight, detected by transducer 30, whereupon pumps 16, 17 and 18 are stopped, clamp 25 opened, clamps 24 and 26 closed, the direction of blood pump 17 reversed, and the red cell rich fraction returned to the donor. The resistance to flow during reinfusion is monitored by the pressure sensor 19. Should the back pressure become excessive the blood pump 17 is stopped. The ultrasonic transducer 20 monitors the donor conduit 9 and reinfusion is stopped if air is detected.

(h) When the weight of red cell chamber 8, detected by transducer 30, falls below a pre-determined minimum, blood pump 17 is stopped and providing the plasma chamber 7 is not full a new blood withdrawal sequence is started, by repeating the sequence (e) to (g) drawing blood and running in anticoagulant as in (b) with the rotor chamber rotating at 1800rpm, harvesting plasma, until a pre-determined weight slightly less than the total required weight of plasma has been collected (ca 500g), detected by transducer 29.

i) Additional plasma is then obtained from the whole blood remaining in the separation chamber 1, without taking further blood from the donor. Pumps 16 and 17 are stopped and, with clamp 26 open and clamps 24 and 25 shut, the red cell pump 18 returns fluid from the red cell reservoir 8 to the separation chamber 1, so displacing plasma into the plasma conduit 4 and hence into the plasma reservoir 7. This process continues until the total required weight of plasma has been collected.

j) The centrifuge rotor then stops, clamp 26 is shut and pump 17 reverses, so pumping the contents of the separation chamber 1 to the red cell reservoir 8. Emptying of the separation chamber 1 is facilitated by the ribs 33, 34 on its internal surface which prevent complete collapse of the separation chamber 1. When the separation chamber 1 is substantially empty (detected by optical transducer 22), the pump 18 stops, the clamp 25 opens and the blood pump 17 returns the contents of the red cell reservoir 8 to the donor. Reinfusion continues until the red cell reservoir 8 is substantially empty.

k)   The donor is disconnected and the plasma reservoir 7 sealed and separated from the remainder of the blood fractionation pathway which is removed from the blood fractionation apparatus and discarded.

The pathway illustrated may be constructed from a variety of bioacceptable materials. However, we particularly prefer the majority of the pathway to be formed from polyvinyl chloride or polycarbonate, with the portion of the pathway from X to Y being of a more torsion stiff, but flexible material, eg nylon. The join between torsion-stiff portion and the remainder of the pathway is preferably at the upper fixed point Y. The two materials may be sealed together using a cyanoacrylate adhesive.

2525Jir/sm

What we claim is:

1.    A blood fractionation system comprising

a centrifugal rotor chamber,

a separation chamber (1) adapted for placement within the rotor chamber,

first and second blood component collection chambers (7, 8 respectively),

first and second blood component conduits (4, 5 respectively) for establishing fluid communication between the separation chamber (1) and the first (7) and second (8) blood component collection chambers respectively,

a donor conduit (9) capable of establishing fluid communication between a donor and the separation chamber (1),

a return conduit (11) for establishing fluid communication between one of the blood component conduits (4, 5) and the donor conduit (9),

a donor pump (17) capable of pumping fluid along the donor conduit (9),

a blood component pump (18) capable of pumping fluid along one of the blood component conduits (4, 5), and

system control means (19 - 31) including means (29, 30) responsive to the weight of fluid in each of the first (7) and second (8) blood component collection chambers, the system control means being adapted to control the system during an operating sequence comprising at least one primary blood component collection phase during which blood is drawn from a donor, the collection phase comprising at least one blood component collection cycle during which blood is continuously drawn from the donor and first blood component is conveyed along its respective blood component conduit (4) whilst flow along the second blood component conduit (5) is stopped, and then second blood component is conveyed along its respective blood component conduit (5) whilst flow along the first blood component conduit (4) is stopped, such that in operation flow of fluid along the first blood component conduit (4) is stopped, and flow of fluid along the second blood component conduit (5) initiated, by the collected first blood component increasing by a pre-determined weight, and flow of fluid along the second blood component conduit (5) is stopped, and flow of fluid along the first blood component conduit (4) initiated, by the collected second blood component increasing by a pre-determined weight.

2. A blood fractionation system according to Claim 1, wherein the means responsive to the weight of fluid in each of the first (7) and second (8) blood component collection chambers includes electronic weight transducers (29, 30).

3. A blood fractionation system according to any one of the preceding claims, wherein the first blood component is a cellular component and the second blood component is a plasma component.

4. A blood fractionation system according to Claim 1, wherein the ratio of the pre-determined weight of collected first blood component to the pre-determined weight of collected second blood component is from 1.7:1 to 2.3:1.

5. A blood fractionation system according to Claim 1, wherein the pre-determined weight increase of collected first blood component is from 0.1 to 20 grams.

6. A blood fractionation system according to any one of the preceding claims, comprising

a disposable blood fractionation pathway (1 - 15) including the separation chamber (1), the first (7) and second (8) blood component collection chambers, the first (4) and second (5) blood component conduits, the donor conduit (9) and the return conduit (11) and

a blood fractionation apparatus (16 - 31) including the centrifugal rotor chamber, the donor pump (17), the blood component pump (18) and the system control means (19 - 31).

7. A blood component fractionation pathway (1 - 15), for use in association with a blood fractionation apparatus including a centrifugal rotor chamber, the pathway comprising

a separation chamber (1) adapted for placement within the rotor chamber of the blood fractionation apparatus,

first (7) and second (8) blood component collection chambers,

first (4) and second (5) blood component conduits for establishing fluid communication between the separation chamber (1) and the first (7) and second (8) blood component collection chambers respectively,

a donor conduit (9) capable of establishing fluid communication between a donor and the separation chamber (1), and

a return conduit (11) for establishing fluid communication between one of the blood component conduits (4, 5) and the donor conduit (9).

8.   A blood fractionation pathway according to Claim 7, wherein the separation chamber (1) is substantially toroidal in shape.

9.   A blood fractionation pathway according to Claim 8, wherein the separation chamber (1) comprises a tube sealed at both ends.

10.   A blood fractionation pathway according to any one of Claims 7 to 9, wherein the first blood component conduit (4) terminates at a position adjacent to the wall (2) of the separation chamber (1) closest to the axis of centrifugation, the second blood component conduit (5) terminates at a position adjacent to the wall (3) of the separation chamber (1) furthest from the axis of centrifugation, and the donor conduit (9) terminates at a position substantially equidistant from said walls (2, 3).

11.  A blood fractionation pathway according to any one of Claims 7 to 10, wherein the first blood component conduit (4) terminates within a blister (6) projecting inwardly from the separation chamber (1) towards the axis of centrifugation.

12.  A blood fractionation pathway according to any one of Claims 7 to 11, wherein the donor conduit (9) is provided with a pressure pouch (14) which is capable of actuating a pressure sensor (19) provided in the blood fractionation apparatus.

13.  A blood fractionation pathway according to any one of Claims 7 to 12, wherein the donor conduit (9) is provided with a filter pouch (15).

14.  A blood fractionation pathway according to any of Claims 7 to 13, wherein the separation chamber (1) is provided on its internal surface with a plurality of longitudinal ribs (33, 34).

15.  A blood fractionation pathway according to Claim 9, wherein the donor conduit (9) is connected to the separation chamber (1) at the opposite end of the separation chamber (1) to the end at which the first (4) and second (5) blood component conduits are connected to the separation chamber (1).

16. A blood fractionation pathway according to any one of Claims 7 to 15, wherein a portion of the pathway including that portion which undergoes angular motion is made of torsion-stiff but flexible material and the remainder of a compliant material.

17. A blood fractionation pathway according to Claim 16, wherein the torsion-stiff but flexible material is nylon and the compliant material is polyvinylchloride.

18. A blood fractionation apparatus for use in association with a blood fractionation pathway according to any one of Claims 7 to 17.

19. A method of fractionating blood comprising separating the blood into first and second blood components in a separation chamber (1) and collecting said first and second blood components in at least one collection cycle during which blood is continuously drawn from a donor, the collection cycle comprising conveying first one and then the other blood component to first (7) and second (8) blood component collection chambers respectively.

20. A method of fractionating blood comprising separating the blood into first and second blood components in a separation chamber (1) and collecting said first and second blood components in first (7) and second (8) blood component collection chambers respectively, wherein the method includes a phase during which no blood is drawn from a donor and one of said first and second blood components is collected by displacing it from the separation chamber (1) by returning the other one of said first and second blood components from its respective blood component collection chamber (7, 8) to the separation chamber (1).

21. A method of fractionating blood comprising separating the blood into first and second blood components in a separation chamber (1) and collecting said first and second blood components in first (7) and second (8) blood component collection chambers respectively, wherein the method includes a final reinfusion phase in which the contents of the separation chamber (1) are conveyed to one of the first (7) and second (8) blood component collection chambers and the contents of that blood component collection chamber are then returned to the donor.

0378K(ir)/ac

Fig.1.

# Fig. 2.